# EUROPEAN PATENT APPLICATION

(11) **EP 4 628 071 A1**
(43) Date of publication of application: **08.10.2025**
(21) Application number: 23896855.6
(22) Date of filing: 30.11.2023
(51) Int. Cl.: A61K 9/16, A61K 35/12, A61K 47/42, A61P 35/00

(54) **TARGETING NANOSCALE PARTICLE, TARGETING CELL, PREPARATION METHOD THEREFOR, AND USE THEREOF**

(30) Priority: 02.12.2022 CN 202211539334
(71) Applicant: Institute of Process Engineering, Chinese Academy of Science, Beijing, 100190 (CN)
(72) Inventor: WEI, Wei, Beijing 100190 (CN); MA, Guanghui, Beijing 100190 (CN); LI, Feng, Beijing 100190 (CN); WANG, Yan, Beijing 100190 (CN); ZHANG, Xiao, Beijing 100190 (CN)
(74) Representative: Niu, Lijiang
(86) International application number: PCT/CN2023/135287
(87) International publication number: WO 2024/114713

(57) **Abstract**

The invention discloses a targeted nanoscale particle, a targeted cell, a preparation method therefor, and use thereof. The targeted nanoscale particle is bound to the outer surface of the targeted cell, and is composed of a plurality of proteins interconnected via a first binding site. The targeted nanoscale particle further comprises a second binding site, and is bound to the outer surface of a target cell via the second binding site. In an exemplary embodiment, the targeted nanoscale particle can promote the interaction between the two types of cells by simultaneously binding to a chimeric antigen receptor T cell and a leukemia cell, thereby promoting the recognition and killing of the leukemia cell by the chimeric antigen receptor T cell. In addition, the internal cavities of the proteins in the targeted nanoscale particle provide space for loading of a chemotherapeutic drug, thus realizing the combination therapy of the chimeric antigen receptor T cell and other therapies while loading the drug.

## Description

### CROSS-REFERENCE TO RELATED APPLICATION

The present application claims priority to application No. CN202211539334.7 entitled "TARGETED NANOPARTICLE, TARGETED CELL, PREPARATION METHOD THEREFOR, AND USE THEREOF" filed on Dec. 2, 2022, the disclosure of which is incorporated herein by reference in its entirety.

### TECHNICAL FIELD

The present invention relates to the technical field of biological immunotherapy, in particular to a targeted nanoparticle, a targeted cell, a preparation method therefor, and use thereof.

### BACKGROUND

Chimeric antigen receptor T cells (CAR T) have achieved remarkable success in the treatment of hematologic malignancies. Currently, CAR T products targeting CD19 have been approved for market release, and multiple CAR T products targeting other antigens are undergoing clinical trials. However, during the progression of leukemia, the expression of target antigens may decrease. Studies have shown that over 30% of B-lineage leukemia patients experience a relapse with reduced CD19 target expression after treatment. The downregulation of target antigen expression severely impairs the ability of CAR T cells to recognize and eliminate leukemia cells, ultimately limiting the therapeutic efficacy of CAR T therapy. Therefore, enhancing the recognition and cytotoxicity of CAR T cells against leukemia cells with low target expression is key to improving CAR T efficacy.

The information provided in BACKGROUND is illustrative only of the general background of the present invention and should not be construed as an acknowledgment or in any way imply that this information forms the prior art, which is well known to those of ordinary skill in the art.

### SUMMARY

The inventors of the present invention found that the ferritin receptor CD71 can be stably and highly expressed on the surfaces of leukemia cells with various disease courses through research, and the expression of CD71 is significantly up-regulated when CAR T cells are expanded *in vitro.* Based on this, the present invention develops a targeted nanoparticle for promoting intercellular interaction based on nanoscale protein particles, thereby promoting the recognition and killing of leukemia cells by, for example, CAR T. In addition, the protein of the present invention has an internal cavity that provides space for loading of chemotherapeutic agents. Thus, the dosage form is also capable of loading drugs to achieve a combination therapy of CAR T with other therapies, ultimately enhancing the efficacy against multiple disease courses of leukemia. Specifically, the present invention includes the following contents.

In one aspect of the present invention, provided is a T cell conjugate, comprising a T cell and a ferritin cluster, wherein the T cell binds to the ferritin cluster, and the ferritin cluster consists of ferritin.

In certain embodiments, provided is the T cell conjugate according to the present invention, wherein the T cell is an activated T cell (typically activated with commercially available activated magnetic beads, or activated with antibodies, etc.), preferably a T cell that has been expanded *in vitro,* and further preferably a freshly prepared chimeric antigen receptor T cell (CAR T cell) or T cell receptor T cell (TCR T cell).

In certain embodiments, provided is the T cell conjugate according to the present invention, wherein the ferritin cluster binds to CD71 of the T cell via receptor-ligand interaction.

In certain embodiments, provided is the T cell conjugate according to the present invention, wherein the average particle size of the ferritin cluster is 50-1500 nm, preferably 300-1500 nm, further preferably 400-1000 nm, and more preferably 500-800 nm.

In certain embodiments, provided is the T cell conjugate according to the present invention, wherein the ferritin cluster is formed by the aggregation and crosslinking of human ferritin; preferably, at least 30% of the exposed ferritin subunits on the surface of the ferritin cluster are heavy chain subunits.

In certain embodiments, provided is the T cell conjugate according to the present invention, wherein the T cell conjugate enhances the targeting to a CD71-expressing target cell via the ferritin cluster.

In certain embodiments, provided is the T cell conjugate according to the present invention, wherein the target cell is primarily a tumor cell, including a solid tumor cell and a hematological tumor cell, such as a cell from liver cancer, lung cancer, colon cancer, cervical cancer, prostate cancer, ovarian cancer, breast cancer, thyroid cancer, esophageal cancer, gastric cancer, and leukemia, preferably a leukemia cell, such as B-ALL, T-ALL, and AML.

The present invention also provides use of the T cell conjugate described above in preparing a medicament for treating or ameliorating a cancer or a tumor associated with CD71 expression, wherein the cancer or tumor is, for example, liver cancer, lung cancer, colon cancer, cervical cancer, prostate cancer, ovarian cancer, breast cancer, thyroid cancer, esophageal cancer, gastric cancer, or leukemia, preferably a leukemia cell, and preferably leukemia.

In one aspect of the present invention, provided is a targeted nanoparticle, wherein the targeted nanoparticle consists of a plurality of proteins interconnected via a first binding site; the targeted nanoparticle further comprises a second binding site, which binds to the outer surface of a target cell via the second binding site.

In certain embodiments, provided is the targeted nanoparticle according to the present invention, wherein the targeted nanoparticle is capable of binding to the outer surface of a targeted cell.

In certain embodiments, provided is the targeted nanoparticle according to the present invention, wherein the first binding site comprises an active group, and the first binding sites are linked by a linker arm.

In certain embodiments, provided is the targeted nanoparticle according to the present invention, wherein the outer surface of the target cell comprises a receptor for the protein, and the receptor is capable of binding to the protein via the second binding site.

In certain embodiments, provided is the targeted nanoparticle according to the present invention, wherein the targeted cell and the target cell are cells that naturally express CD71, wherein the targeted cell comprises at least one of T cells, NK cells, B cells, lymphocytes, cytokine-induced killer cells, natural lymphoid cells, and/or leukocytes.

In certain embodiments, provided is the targeted nanoparticle according to the present invention, wherein the type of the T cells is not particularly limited; preferably, the T cells comprise modified and unmodified T cells, wherein the modified T cells comprise CAR T cells and TCR T cells, and the unmodified T cells comprise tumor infiltrating T cells, PBMC-derived T cells, or other T cells isolated *in vivo.*

In certain embodiments, provided is the targeted nanoparticle according to the present invention, wherein the targeted nanoparticle is 50 nm or more and 5000 nm or less, preferably 100 nm or more and 3000 nm or less, further preferably 300-2000 nm, and more preferably 300-1000 nm.

In certain embodiments, provided is the targeted nanoparticle according to the present invention, wherein the protein comprises an internal cavity.

In certain embodiments, provided is the targeted nanoparticle according to the present invention, wherein the internal cavity contains or does not contain a drug.

In certain embodiments, provided is the targeted nanoparticle according to the present invention, wherein the protein is selected from at least one of natural ferritin, recombinant full-heavy chain ferritin, and genetically engineered ferritin.

In one aspect of the present invention, provided is a method for preparing the targeted nanoparticle described above, comprising:
(1) aggregating and precipitating the protein to form a protein cluster under electrolyte or non-electrolyte conditions; and
(2) crosslinking the protein clusters in the presence of a crosslinking agent to obtain a crosslinked targeted nanoparticle.

In certain embodiments, provided is the method for preparing the targeted nanoparticle according to the present invention, wherein the type of the crosslinking agent is not particularly limited as long as it can crosslink the protein clusters, examples of which include, but are not limited to: glutaraldehyde, NHS-PEGₓ-NHSDSS, disuccinimidyl suberate, paraformaldehyde, and polyethyleneimine.

In certain embodiments, provided is the method for preparing the targeted nanoparticle according to the present invention, wherein the method optionally comprises a step of introducing an immunotherapeutic drug and/or a small molecule chemical drug into the cavity of the protein before step (1).

In one aspect of the present invention, provided is a targeted cell, wherein the outer surface of the targeted cell is bound to the targeted nanoparticle according to the first aspect; a plurality of proteins are interconnected via a first binding site to form the targeted nanoparticle, the targeted nanoparticle further comprises a second binding site, and binds to the outer surface of a target cell via the second binding site.

In certain embodiments, provided is the targeted cell according to the present invention, wherein the first binding site comprises an active group, and the first binding sites are linked by a linker arm.

In certain embodiments, provided is the targeted cell according to the present invention, wherein the outer surface of the target cell comprises a receptor for the protein, and the receptor is capable of binding to the protein via the second binding site.

In certain embodiments, provided is the targeted cell according to the present invention, wherein the targeted cell and the target cell are cells that naturally express CD71.

In certain embodiments, provided is the targeted cell according to the present invention, wherein the cell is selected from at least one of αβT cells, γδT cells, natural killer (NK) cells, natural lymphoid cells (ILC), cytokine-induced killer (CIK) cells, cytotoxic T lymphocytes (CTL), lymphokine-activated killer (LAK) cells, T lymphocytes, and peripheral blood mononuclear cells. Preferably, the targeted cell is selected from at least one of CAR T cells, TCR T cells, and tumor infiltrating lymphocytes; the target cell includes cells from a hematologic tumor, a solid tumor, or a combination thereof.

In certain embodiments, provided is the targeted cell according to the present invention, wherein the targeted nanoparticle is 50 nm or more and 5000 nm or less, preferably 300 nm or more and 3000 nm or less.

In certain embodiments, provided is the targeted cell according to the present invention, wherein the protein comprises an internal cavity.

In certain embodiments, provided is the targeted cell according to the present invention, wherein the internal cavity contains or does not contain a drug.

In certain embodiments, provided is the targeted cell according to the present invention, wherein the protein is selected from at least one of natural ferritin, recombinant full-heavy chain ferritin, and genetically engineered ferritin.

In one aspect of the present invention, provided is a method for preparing a targeted cell, comprising:
(1) aggregating and precipitating ferritin to form a protein cluster under electrolyte or non-electrolyte conditions,
(2) crosslinking the clusters in the presence of a crosslinking agent to obtain a crosslinked targeted nanoparticle;
(3) enabling the targeted nanoparticle and a cell to be close to and in contact with each other to obtain the targeted cell.

In certain embodiments, provided is the preparation method according to the present invention, wherein optionally, an immunotherapeutic drug and/or a small molecule chemical drug is introduced into the cavity of the protein before step (1).

In one aspect of the present invention, provided is a method for promoting the proximity of cells to each other, comprising a step of using the targeted nanoparticle to promote cell approach and contact, wherein a plurality of proteins are interconnected via a first binding site to form the targeted nanoparticle, the targeted nanoparticle further comprises a second binding site, and binds to the outer surface of the cell via the second binding site.

In certain embodiments, provided is the method according to the present invention, wherein the method is an *in vitro* method, and the cell comprises a first cell and a second cell. The first cell and the second cell may be the same cell or different cells. Preferably, the first cell and the second cell are different. Also preferably, the surface of the first cell is bound to the targeted nanoparticle via, for example, CD71, and the surface of the second cell has CD71.

In certain embodiments, provided is the method according to the present invention, wherein the cell is selected from at least one of CAR T cells, TCR T cells, and tumor infiltrating lymphocytes, or a cancer or tumor cell, such as a leukemia cell, wherein the cancer or tumor comprises a hematologic tumor, a solid tumor, or a combination thereof. In certain embodiments, provided is the method according to the present invention, wherein the hematological tumor is selected from the group consisting of: acute myeloid leukemia (AML), multiple myeloma (MM), chronic lymphocytic leukemia (CLL), acute lymphoblastic leukemia (ALL), diffuse large B-cell lymphoma (DLBCL), and a combination thereof.

In certain embodiments, provided is the method according to the present invention, wherein the solid tumor is selected from the group consisting of: gastric cancer, gastric cancer peritoneal metastasis, liver cancer, leukemia, kidney tumor, lung cancer, small intestine cancer, bone cancer, prostate cancer, colorectal cancer, breast cancer, large intestine cancer, cervical cancer, ovarian cancer, lymph cancer, nasopharyngeal cancer, adrenal gland tumor, bladder tumor, non-small cell lung cancer (NSCLC), brain glioma, cervical cancer, endometrial cancer, mesothelioma, pancreatic cancer, and a combination thereof.

In one aspect of the present invention, provided is a pharmaceutical composition, comprising the targeted nanoparticle or the targeted cell according to the present invention.

In certain embodiments, provided is the pharmaceutical composition according to the present invention, further comprising an immunotherapeutic drug and/or a small molecule chemical drug.

In one aspect of the present invention, provided is a use of the targeted nanoparticle or the targeted cell according to the present invention, or the pharmaceutical composition in the manufacture of a medicament for treating a cancer or a tumor, wherein the cancer or the tumor comprises CD71 positive tumor or cancer, preferably B-lineage leukemia.

In one aspect of the present invention, provided is a use of the targeted nanoparticle or the targeted cell according to the present invention, or the pharmaceutical composition in combination with other drugs. Other drugs include, but are not limited to, diagnostic, prophylactic and/or therapeutic agents.

Technical effects of the present invention include, but are not limited to:
In the exemplary embodiments of the present invention, a ferritin cluster nanoparticle formulation is prepared using the aggregation and precipitation-crosslinking method. It has been found that this formulation can modify the surface of CAR T by binding to CD71 on the CAR T surface. Due to the relatively large particle size of the clusters, it avoids uptake of ferritin clusters by CAR T cells, which have weak endocytosis capability, ensuring the long-term effective retention of ferritin clusters on the CAR T surface. The modified CAR T cells enhance intercellular interactions by binding the surface ferritin clusters to CD71 on the surface of leukemia cells, thereby promoting the recognition of leukemia antigen targets by CAR T cells and further enhancing CAR T cell activation. Furthermore, the strong endocytosis ability of leukemia cells allows them to take up ferritin clusters from the CAR T surface. This process facilitates the targeted cytotoxicity of the drugs in the ferritin cavity against leukemia cells, ensuring the combined treatment of leukemia by CAR T therapy and other therapeutic approaches.

In the present invention, it is further discovered that the nano-protein particle possesses an internal cavity capable of loading various drugs, such as arsenic trioxide, doxorubicin, and ferrosoferric oxide, enabling the combination of ferritin-based therapy with multiple other treatment modalities. Additionally, ferritin can be genetically engineered to expand the cavity volume or modify internal amino acid residues, allowing for a greater variety and quantity of drug loading, thus enabling more therapeutic strategies in combination with CAR T therapy.

### BRIEF DESCRIPTION OF THE DRAWINGS

FIG. 1 shows a flow diagram of an exemplary CAR-T cell therapy for leukemia.
FIG. 2 shows a transmission electron micrograph of exemplary ferritin and ferritin clusters.
FIG. 3 shows atomic force microscope measurement results showing the force between an exemplary CAR T and leukemia cells.
FIG. 4 shows the proliferation of different CAR T after co-incubation with leukemia cells measured using the CFSE method.
FIG. 5 shows positive proportions of granzyme B and interferon γ (IFNγ) after co-incubation of different CAR T with leukemia cells by flow cytometry.
FIG. 6. shows killing results of CAR T and FnC-CAR T incubated with leukemia cells at different ratios.
FIG. 7 shows the results of detecting the change of peripheral blood leukemia load over time after CAR T, FnC-CAR T, and high dose CAR T were reinfused into leukemia model mice.
FIG. 8 shows atomic force microscope measurement results showing the force between CAR T and FnC-CAR T and leukemia cells with low expression of CD19.
FIG. 9 shows the results of proliferation assay after incubation of CAR T and FnC-CAR T with leukemia cells with low expression of CD19.
FIG. 10 shows activation of CAR T after co-incubation with leukemia cells with low expression of CD19 as detected by flow cytometry.
FIG. 11 shows the results of the killing assay of CAR T on leukemia cells with low expression of CD19.
FIG. 12 shows the results of detecting the change of peripheral blood leukemia load over time after CAR T, FnC-CAR T, and high dose CAR T were reinfused into model mice of leukemia cells with low expression of CD19.
FIG. 13 shows leukemia cells capturing FnC from the surface of CAR T cells observed by dynamic fluorescence imaging.
FIG. 14 shows the results of detecting the change of peripheral blood leukemia load over time after CAR T, FnC-CAR T, and FnC-CAR T loaded with arsenic trioxide were reinfused into high-load CD19 low-expression leukemia model mice from different treatment starting points.
FIG. 15 shows the force between tumor infiltrating lymphocytes and FnC-TIL and tumor cells and the activation condition of immune cells.
FIG. 16 shows the force between FnC-TCR T and tumor cells and the activation condition of immune cells.
FIG. 17 shows the force between FnC-^{EGFR}CAR T and tumor cells and the activation condition of immune cells.
FIG. 18 shows that expression of CD71 appears to be greatly up-regulated when CAR T cells are expanded *in vitro,* where Rest T refers to newly isolated T cells from human PBMCs without any additional manipulation; CAR T refers to CAR T after preparation and *in vitro* expansion using T cells isolated from human PBMCs.
FIG. 19 shows the statistical results of killing of bone marrow samples from clinically different leukemia patients by different CAR T cells.
FIG. 20 shows the results of detecting the change of peripheral blood leukemia load over time after CAR T, FnC-CAR T, and high dose CAR T were reinfused into T-ALL leukemia PDX model mice.
FIG. 21 shows the results of detecting the change of peripheral blood leukemia load over time after CAR T, FnC-CAR T, and high dose CAR T were reinfused into CD7 low-expression T-ALL leukemia PDX model mice.
FIG. 22 shows the results of detecting the change of peripheral blood leukemia load over time after FnC-CAR T and FnAsC-CAR T were reinfused into high-load CD7 low-expression T-ALL leukemia PDX model mice.

### DETAILED DESCRIPTION

Various exemplary embodiments of the present invention are described in detail below. The detailed description should not be construed as limitations on the present invention but as a more detailed description of certain aspects, features, and embodiments of the present invention. Examples where the specific technologies or conditions are not specified are performed according to the technologies or conditions described in the publications of the art (e.g., see, *Molecular Cloning: A Laboratory Manual,* authored by J. Sambrook et al., and translated by Huang Peitang et al., third edition, Science Press) or according to the package insert. Reagents or instruments without specified manufacturers used herein are conventional products that are commercially available.

It will be appreciated that the terms used herein are for the purpose of illustrating particular embodiments only, rather than limiting the present invention. In addition, for the numerical ranges in the present invention, it will be appreciated that the upper and lower limits of the ranges are specifically disclosed, as well as every intervening value between them. Every smaller range between any stated value or intervening value in a stated range and any other stated or intervening value in that stated range is encompassed within the present invention. The upper and lower limits of these smaller ranges may independently be included or excluded in the range.

Unless defined otherwise, all technical and scientific terms used herein have the same meaning as commonly understood by those of ordinary skill in the art to which the present invention pertains. Although only preferred methods and materials are described herein, any methods and materials similar or equivalent to those described herein can be used in the practice or testing of the present invention. All documents described herein are incorporated by reference to disclose and describe the methods and/or materials associated with the documents. In case of conflict with any incorporated document, the specification shall prevail.

### Targeted cell

The targeted cell of the present invention can significantly kill tumor cells with surface expression of CD71 or CD71 positive tumors, so that the targeted cell can be applied to treating or ameliorating diseases related to CD71 expression, laying the foundation for treating CD71 positive tumors.

In the present invention, a "targeted cell" is an immune cell that can perform immune effector functions. Preferably, the immune cell is selected from: at least one of immune cells, T lymphocytes or modified T cells, NK cells, peripheral blood mononuclear cells (PBMCs), and hematopoietic stem cells, which are cultured and differentiated from pluripotent stem cells or embryonic stem cells. More preferably, the immune cell is a T lymphocyte (sometimes abbreviated herein as a T cell). Further preferably, the immune cell is a modified T cell, i.e. a CAR-T cell or a TCR-T cell. In some embodiments, the T cell may be CD4+/CD8-, CD4-/CD8+, CD4+/CD8+, CD4-/CD8-, or a combination thereof. In some embodiments, T cells produce IL-2, IFN, and/or TNF when expressing a chimeric antigen receptor and binding to a target cell. In some embodiments, CD8+ T cells lyse antigen-specific target cells when expressing a chimeric antigen receptor and binding to a target cell.

In the present invention, CD71 is expressed on the surface of the T cell, and the targeted nanoparticle of the present invention can bind thereto, wherein the targeted nanoparticle comprises a nanoscale particle or a polymeric structure, which is formed by a plurality of ferritin. "Binding" and "immunoreaction therewith" or "targeting" are used interchangeably to refer to a non-covalent interaction that occurs between an immunoglobulin molecule and an antigen specific for the immunoglobulin.

The ferritin of the present invention may be natural ferritin, or recombinant full-heavy chain ferritin or genetically engineered ferritin, which is not particularly limited. The specific sequence of ferritin is not particularly limited so long as it has biological activity or binding properties that target the ferritin receptor CD71 or it is a ferritin with improved affinity, and such other variants can be obtained using methods known in the art and are included within the scope of the present invention. The amino acid sequence of a polypeptide or protein can be modified by a person skilled in the art using recombinant methods and/or synthetic chemical techniques for producing variant polypeptides or proteins. For example, amino acid substitutions or modifications can be used to obtain ferritin with further improved affinity.

The term "modified" as used herein means that amino acid modifications do not significantly affect or alter the binding characteristics of ferritin. Such modifications include amino acid substitutions, additions, and deletions. Preferably, residue positions that are not identical differ by conservative amino acid substitutions. Ferritin of the present invention may include glycosylation, acetylation, phosphorylation, amidation, derivatization by known protecting/blocking groups, proteolytic cleavage, or non-naturally occurring amino acid modifications, and the like. In the present invention, a plurality of proteins are connected with each other via a first binding site to form the targeted nanoparticle, the first binding site comprises an active group, and the first binding sites are covalently linked by a linker arm. Examples of active groups include, but are not limited to, -COOH, -NH₂, and -HS. The linker arm comprises an aldehyde group and/or a short chain small molecule of a polyethylene glycol derivative. In certain embodiments, the aldehyde group-containing compound comprises glutaraldehyde. Preferably, the polyethylene glycol derivative is NHS-PEGₓ-NHS, wherein the subscript x represents multiple repeating units of the polyethylene glycol. Also preferably, x is an integer from 1 to 100.

In certain embodiments, a plurality of ferritin can form nanoscale particles or polymeric structures, and the ferritin has a cavity sufficient for accommodating an optional drug or small molecule chemical drug, and the particle size of the formed targeted nanoparticles is 50-1500 nm, preferably 100 nm or more and 1500 nm or less, further preferably 300-1500 nm, further preferably 300-800 nm, such as 320 nm, 340 nm, 360 nm, 380 nm, 400 nm, 420 nm, 440 nm, 460 nm, 480 nm, 500 nm, 600 nm, 700 nm, 800 nm or any value range therebetween. The particle size enables the CAR-T cell to have relatively weak uptake capacity on the nanoscale particles, and ensures that the nanoscale particles are modified on the surface of the CAR-T cell. More importantly, this particle size range enables the target cell to capture the nanoscale particle from the CAR-T cell by its endocytosis. In addition, the targeted nanoparticles of the present invention can promote the interaction between cells. In an exemplary embodiment, the targeted nanoparticle can bind to a chimeric antigen receptor T cell and a leukemia cell at the same time to promote the interaction between the two cells, thereby promoting the recognition and killing of the leukemia cell by the chimeric antigen receptor T cell.

In certain embodiments, the target cell includes a cancer or a tumor cell from a cancer or tumor patient, a patient suspected of having cancer or tumor, or a cancer or tumor patient undergoing treatment, preferably a positive tumor that expresses the ferritin receptor CD71 on the cell surface. Also preferably, the target cell is a leukemia cell.

In the present invention, the targeted nanoparticle further comprises a second binding site, and binds to the outer surface of the target cell through the second binding site. The outer surface of the target cell expresses ferritin receptor CD71, and the ferritin receptor CD71 can bind to the protein through the second binding site. Preferably, the second binding site is different from the first binding site.

### Preparation method

The present invention further provides a preparation method for a targeted cell, which comprises steps (1) to (3). In step (1), allow the protein to aggregate and precipitate to form a protein cluster under electrolyte or non-electrolyte conditions, and the electrolyte condition refers to adding an electrolyte solution into the ferritin solution. The electrolyte solution is not particularly limited, and in certain embodiments, a saturated ammonium sulfate solution is used. The non-electrolyte condition means that a non-electrolyte solution is added to the ferritin solution. The non-electrolyte solution is not particularly limited, and in certain embodiments, the non-electrolyte solution is ethanol. The purpose of step (1) is to aggregate and precipitate ferritin to form ferritin clusters, and therefore the electrolyte and non-electrolyte related reagents only need to be able to disrupt the stability of the protein solution. It will be appreciated by those skilled in the art that, for example, ethanol and saturated ammonium sulfate solutions may be used simultaneously or separately to form ferritin clusters.

In step (2), crosslink the clusters in the presence of a crosslinking agent to obtain a crosslinked targeted nanoparticle. Preferably, the crosslinking agents include glutaraldehyde and NHS-PEGₓ-NHS. It is understood that other crosslinking agents may be used by those skilled in the art to enable crosslinking of a plurality of ferritin to form aggregates or clusters having a certain particle size. Such crosslinking agents further include: disuccinimidyl suberate, paraformaldehyde, and polyethyleneimine. After step (2), a cleaning step is further included to remove excess reactants.

In step (3), enable the targeted nanoparticle and a cell to be close to and in contact with each other, such that ferritin nanoclusters can be modified on the CAR-T surface by binding to CD71 to obtain the targeted cell. Preferably, enabling the targeted nanoparticle and a cell to be close to and in contact with each other is performed under *ex vivo* conditions.

It will be appreciated by those skilled in the art that in order to achieve the combination administration or combination therapy, a step of introducing an immunotherapeutic drug and/or a small molecule chemical drug into the cavity of the protein is further included before step (1), and therefore, additional drug loading steps are also within the scope of the present invention. The specific steps of drug loading are not particularly limited, and different drug loading methods can be used according to the type of the drug. For example, the drug can be loaded before forming clusters, or the drug can be loaded through dissociation self-assembly, or the drug can be loaded directly through the pore of ferritin.

### Method for promoting cell proximity or binding

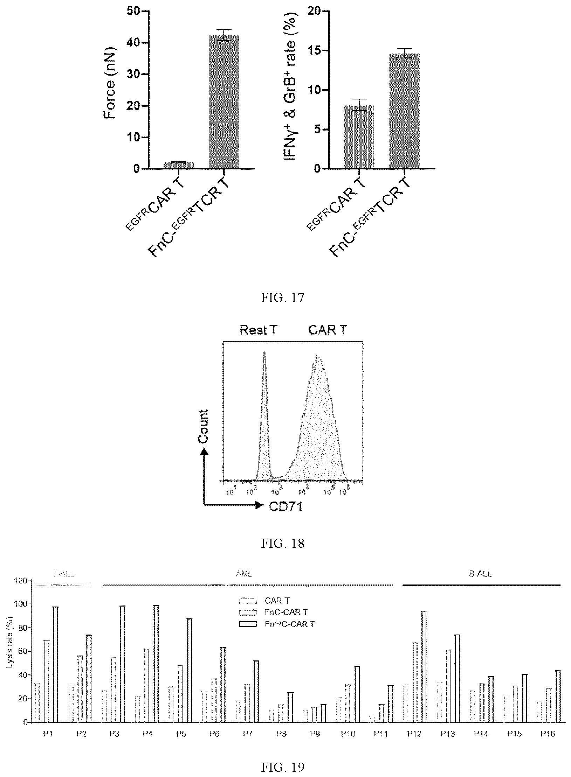

The present invention further provides a method for promoting cell proximity or binding, comprising the step of using the targeted nanoparticle to promote cell proximity and contact. Preferably, enabling the targeted nanoparticle and a cell to be close to and in contact with each other is performed under *ex vivo* conditions. It is noted that the cells herein include a first cell and a second cell, wherein the first cell is selected from at least one of CAR T cells, TCR T cells, and tumor infiltrating lymphocytes. The second cell is selected from a cancer or tumor cell. In certain embodiments, the targeted nanoparticle binds to CD71 on the cell surface of at least one of a CAR T cell, a TCR-T cell, a tumor infiltrating lymphocyte to form a conjugate, and the conjugate binds to CD71 on the surface of a cancer or tumor cell via the targeted nanoparticle. In this process, the inventors found that the expression of CD71 appears to be significantly up-regulated during the *in vitro* construction and expansion process of CAR-T (see FIG. 18). By co-incubation and binding of the targeted nanoparticle with the CAR T, the intercellular forces are greatly enhanced, the recognition of cancer or tumor antigen targets by the CAR-T cells is promoted, and in turn the activation of the CAR-T cells is promoted. Therefore, the modified CAR-T cell of the present invention can effectively solve the problem that the target antigen expression down-regulation seriously influences the recognition and killing of leukemia cells by CAR T cells, and further the modified CAR-T cell of the present invention can remarkably promote the recognition and killing of the low-target leukemia cells by CAR T.

### Pharmaceutical composition

The present invention also provides a pharmaceutical composition comprising the targeted cell as described herein. In some embodiments, the pharmaceutical composition further comprises a pharmaceutically acceptable carrier. Pharmaceutical compositions can be prepared by mixing the active agent of the desired purity with an optional pharmaceutically acceptable carrier in the form of a lyophilized formulation or an aqueous solution. Pharmaceutically acceptable carriers are non-toxic to recipients at the dosages and concentrations employed and may include at least one of buffers, antioxidants, preservatives, isotonicity agents, stabilizers, and surfactants. Furthermore, in order for pharmaceutical compositions to be useful for *in vivo* administration, they must be sterile. The pharmaceutical composition may be sterilized by filtration through a sterile filtration membrane.

In some embodiments, the pharmaceutical composition may contain: at least one additive of cytotoxic agents, chemotherapeutic agents, cytokines, immunosuppressive agents, growth inhibitory agents, and active agents required for the particular indication being treated. The specific addition amount of the additive can be adjusted according to actual needs.

### Use

The present invention also provides a use of an agent comprising the targeted cell or the pharmaceutical composition as described herein in the manufacture of a medicament for treating or ameliorating cancer.

Preferably, the treatment or amelioration of a cancer or a tumor refers to the ability to stimulate or enhance immune function in a patient with the cancer or the tumor.

Preferably, the cancer or the tumor is a cancer associated with CD71 expression.

The term "cancer or tumor associated with CD71 expression" as used herein refers to a disease caused directly or indirectly by expression of CD71 by cells, preferably a cancer or a tumor with high or excessive expression of CD71. Preferably, the cancer or the tumor comprises a hematologic cancer or tumor, a solid tumor, or a combination thereof.

Hematologic cancers are cancers of the blood or bone marrow. Examples of hematologic (or hematological) cancers include leukemia, including acute leukemia (such as acute lymphocytic leukemia, acute myelogenous leukemia, acute myelogenous leukemia, and myeloblastic, promyelocytic, granulo-monocytic, monocytic, and erythroleukemia), chronic leukemia (such as chronic myelogenous (granulocytic) leukemia, chronic myelogenous leukemia, and chronic lymphocytic leukemia), polycythemia vera, lymphoma, Hodgkin's disease, non-Hodgkin's lymphoma (indolent and higher order forms), multiple myeloma, waldenstrom's macroglobulinemia, heavy chain disease, myelodysplastic syndrome, hairy cell leukemia, and myelodysplasia.

A solid tumor is an abnormal mass of tissue that generally does not contain cysts or fluid areas. Solid tumors can be benign or malignant. Different types of solid tumors are named for the type of cells that form them (such as sarcomas, carcinomas, and lymphomas). Examples of solid tumors such as sarcomas and carcinomas include fibrosarcoma, myxosarcoma, liposarcoma mesothelioma, lymphoid malignancies, pancreatic cancer, and ovarian cancer.

The present invention therefore also provides a method for treating /and preventing cancer comprising the step of administering to a subject in need a therapeutically effective amount of a targeted cell or a pharmaceutical composition. As used herein, the terms "subject" and "patient" are used interchangeably herein to refer to any animal that may be in need of treatment with the targeted cell or the pharmaceutical composition described herein. Subjects and patients thus include, but are not limited to: primates (including humans), canines, felines, murines, and other mammalian subjects. Preferably, the subject is a human.

In the present invention, the term "treatment" refers to both therapeutic treatment and prophylactic or preventative measures, the object of which is to prevent or slow down (lessen) the progression of an undesired physiological change or disorder, such as an immune disease of the blood system. Beneficial or desired clinical results include, but are not limited to, relieved symptoms, diminished extent of disease, stabilized (i.e., not worsening) state of disease, delayed or slowed disease progression, ameliorated or palliated state of disease, and alleviation (partial or total), whether detectable or undetectable. "Treatment" is also intended to refer to an extended survival compared to the survival expected for those not receiving the treatment. Those in need of the treatment include those with the condition or disorder, as well as those susceptible to the condition or disorder, or those in whom the condition or disorder is to be prevented.

As used herein, the term "effective amount" refers to an amount of a drug or an agent that elicits, for example, a biological or pharmaceutical response in a tissue, a system, an animal, or a human, as sought by a researcher or a clinician. In addition, the term "therapeutically effective amount" refers to an amount that causes improved treatment, cure, prevention, or alleviation of a disease, a disorder, or a side effect, or a decrease in a rate of progression of a disease or a condition, as compared with a corresponding subject not receiving that amount. The term further includes within its scope an amount effective to enhance normal physiological function. In general, the effective amount herein will vary depending on various factors, such as the given drug or compound, pharmaceutical formulation, route of administration, type of disease or disorder, subject being treated, and the like, but can nevertheless be routinely determined by a person skilled in the art. An effective amount of the targeted cell or the pharmaceutical composition of the present invention can be readily determined by one skilled in the art by routine methods known in the art.

Administration of the targeted cell or the pharmaceutical composition of the present invention can be performed in any convenient manner, including by nebulization, injection, swallowing, infusion, implantation, or transplantation. The targeted cell or the pharmaceutical composition described herein can be administered to a patient subcutaneously, intradermally, intratumorally, intranodal, intraspinal, intramuscularly, by intravenous (i.v.) injection, or intraperitoneally. In certain embodiments, the T cell composition of the present invention is administered to a patient by intradermal or subcutaneous injection. In another embodiment, the T cell composition of the present invention is preferably administered by i.v. injection. Compositions comprising T cells may be injected directly into a tumor, lymph node, or site of infection.

### Combination therapy

The present invention also provides use of the targeted cell or the pharmaceutical composition according to the present invention in combination with other drugs. Preferably, the other drugs include detection reagents, prophylactic agents and/or therapeutic agents. Also preferably, the other drugs are immunotherapeutic drugs and/or small molecule chemical drugs, examples of which include, but are not limited to: chemotherapeutic drugs, radioisotopes, cytokines, nucleic acids, anti-tumor or anti-inflammatory drugs. The drug can be loaded into the ferritin internal cavity of the targeted nanoparticle of the present invention, or administered before, after, or simultaneously with the administration of targeted cells carrying the targeted nanoparticle, whether or not they are loaded with a drug.

### Example 1

### 1. Material

Full-heavy chain recombinant ferritin (expressed by engineering bacteria), PBS buffer, deionized water, absolute ethanol, ammonium sulfate, glutaraldehyde, NHS-PEG_{X}-NHS (Sigma), polylysine (Sigma), Cell-Tak adhesive (Corning), CFSE dye (Sigma), flow cytometry intracellular staining set (Becton, Dickinson and Company), human CD3 flow cytometry antibody (Biolegend), human IFNγ flow cytometry antibody (Biolegend), human granzyme B flow cytometry antibody (Biolegend), human CD19 flow cytometry antibody (Biolegend), red blood cell lysis buffer (Solarbio), and LDH release detection kit (Solarbio).

### 2. Method

The preparation of the targeted cell of the present invention comprises the following steps:
(1) The protein was aggregated and precipitated to form a protein cluster under electrolyte or non-electrolyte conditions,
   non-electrolyte conditions: Ferritin was dissolved in PBS buffer (pH 7.2-7.4) to a final concentration of 10 mg/mL. Then, absolute ethanol was added dropwise to the solution at room temperature under magnetic stirring at 1000 rpm until the solution became cloudy visible to the naked eye.
   Electrolyte conditions: Ferritin was dissolved in PBS buffer (pH 7.2-7.4) to a final concentration of 10 mg/mL. Then, a saturated ammonium sulfate solution was added dropwise to the solution at room temperature under magnetic stirring at 1000 rpm until the solution became cloudy visible to the naked eye.
(2) The clusters were crosslinked in the presence of a crosslinking agent to obtain a crosslinked targeted nanoparticle. The crosslinking in the step was to directly add a 25% glutaraldehyde solution into the turbid solution in step (1) until the final concentration was 0.25-1%, or add NHS-PEG-NHS to the final concentration of about 1%. The mixture was then allowed to react for 1.5 h at room temperature with stirring at 1000 rpm. After the reaction was completed, PBS buffer was added to the system at room temperature under stirring at 1000 rpm, and the system was diluted to 2 times the original volume. Finally, the system was centrifuged at 10000 g for 10 min, and the supernatant was discarded. The precipitate was washed 3 times with PBS buffer to obtain the ferritin cluster nano-particle formulation. The clusters were stored in PBS buffer.
(3) The targeted nanoparticle and a cell was enabled to be close to and in contact with each other to obtain the targeted cell: CAR T cells and the ferritin cluster nano-particle formulation were co-incubated for 1 h according to the ratio of 0.5 mg ferritin cluster/10⁶ CAR T, and the mixture was centrifuged and washed to remove redundant ferritin clusters. 0.5 mg of ferritin clusters was used based on the protein mass thereof.

### 3. Force detection

The force between CAR T and FnC-CAR T and leukemia cells was detected using an atomic force microscope.

Firstly, a 6 cm-diameter culture dish was coated with polylysine and incubated, then leukemia cells were cultured in the culture dish, and the leukemia cells were adsorbed to the bottom of the culture dish by utilizing the polylysine. CAR T or FnC-CAR T was then adhered to the probe of an atomic force microscope using Cell-Tak adhesive, and the force between CAR T or FnC-CAR T on the probe and the leukemia cells was then detected using the atomic force microscope.

### 4. Proliferation assay after incubation of CAR T and FnC-CAR T with leukemia cells

Proliferation of CAR T after co-incubation with leukemia cells was measured using the CFSE method. CFSE-stained FnC-CAR T was constructed by using CFSE dye to stain CAR T, and then incubating the partially stained CAR T with FnC. CFSE-stained CAR T and FnC-CAR T were then separately co-incubated with leukemia cells. Finally, CFSE fluorescence signals of CAR T or FnC-CAR T were detected using flow cytometry after co-incubation for 0 h, 24 h, and 72 h, respectively, to detect both proliferation status (where CAR T or FnC-CAR T was labeled with a human CD3 antibody).

### 5. CAR T and FnC-CAR T activation by co-incubation with leukemia cells

CAR T and FnC-CAR T were activated by co-incubation with leukemia cells as detected by flow cytometry.

The positive ratio of IFN-γ to granzyme B after CAR T and FnC-CAR T co-incubation with leukemia cells was tested using IFN-γ and granzyme B antibodies according to the intracellular staining protocol provided by Becton, Dickinson and Company to characterize the extent of activation (where CAR T or FnC-CAR T was labeled with a human CD3 antibody).

### 6. Killing of CAR T and FnC-CAR T incubated with leukemia cells at different ratios

Killing of leukemia cells by CAR T was characterized by detecting supernatant LDH release.

CAR T or FnC-CAR T was blended with leukemia cells at different ratios and then cultured in 96-well plates. After 24 h, the cell mixture was taken out and centrifuged, and the supernatant was taken. LDH release in the supernatant was detected using an LDH release detection kit (Solarbio), and the ratio of the leukemia cells lysed was characterized by comparison with the positive control group.

### 7. Change of peripheral blood leukemia load over time

Peripheral blood leukemia load was measured as a function of time following reinfusion of CAR T, FnC-CAR T, and high dose CAR T into leukemia model mice.

Firstly, leukemia modeling: Leukemia cells isolated from bone marrow of clinical leukemia patients were reinfused intravenously into severely immunodeficient mice at a dose of 5 × 10⁶ cells/mouse (NTG mice, SPF company). After about 7 days of reinfusion, orbital blood of the mouse was sampled, and the proportion of the human leukemia cells in the white cells of the sample of the blood of the mouse was detected by flow cytometry. When the proportion was not less than 1%, it was determined that the modeling of the leukemia was successful.

Treatment regimen: After the leukemia model was successfully constructed, the model mice were randomly grouped (no less than 8 mice per group), and then PBS buffer (200 µL/mouse), CAR T (1 × 10⁶/mouse), FnC-CAR T (1 × 10⁶/mouse), and high dose CAR T (5 × 10⁶/mouse) were intravenously reinfused into the model mice. After reinfusion, the proportion of the human leukemia cells in the mouse peripheral blood white cells was detected once every 5 days so as to detect the leukemia load change of the model mouse after the treatment.

### 2. Experimental results

### 2.1 Ferritin and ferritin clusters

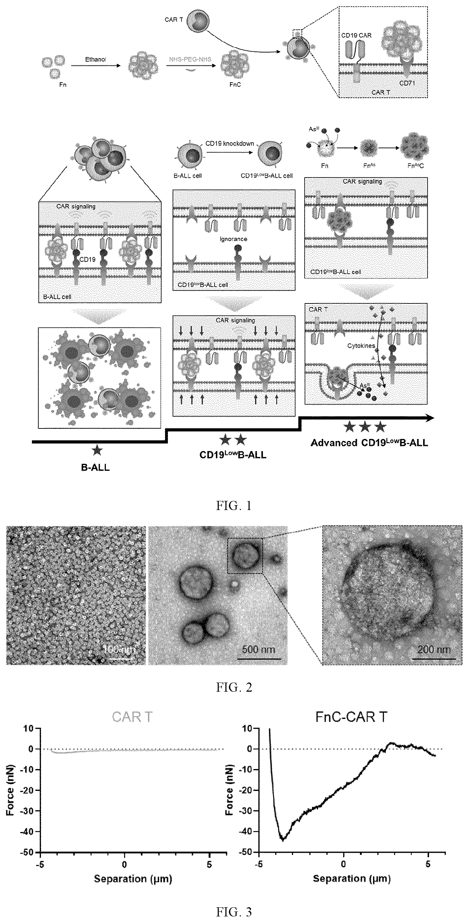

FIG. 1 shows a general schematic diagram of the modified CAR-T cell of the present invention for leukemia therapy. The transmission electron microscopy of the prepared ferritin and ferritin cluster is shown in FIG. 2. Ferritin cluster with a particle size of about 300-500 nm was successfully constructed according to the method of the present invention.

### 2.2 Force detection

The force between CAR T and leukemia cells was measured by an atomic force microscope. From FIG. 3, it can be seen that after FnC modification, the force between CAR T and leukemia cell significantly increased (from about 2 nN to about 43 nN).

### 2.3 Proliferation of CAR T after co-incubation with leukemia cells measured using the CFSE method

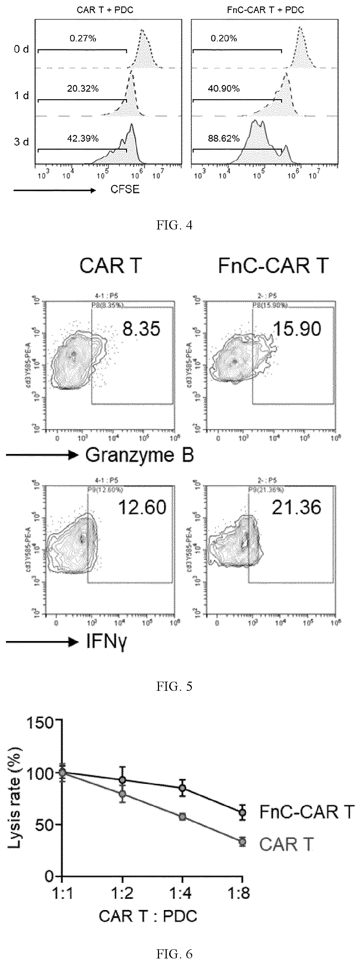

As can be seen in FIG. 4, after FnC modification, CAR T proliferated significantly after co-incubation with leukemia cells.

### 2.4 Activation of CAR T and FnC-CAR T by co-incubation with leukemia cells as detected by flow cytometry

FIG. 5 shows positive proportions of granzyme B and interferon γ (IFN-γ) after co-incubation of CAR T with leukemia cells by flow cytometry to characterize CAR T activation. As can be seen in FIG. 5, after FnC modification, the activation proportion of CAR T after co-incubation with leukemia cells was significantly increased.

### 2.5 Killing of CAR T and FnC-CAR T incubated with leukemia cells at different ratios

FIG. 6 shows killing of CAR T on leukemia cells by detecting supernatant LDH release, with killing ratio on the ordinate. As can be seen in FIG. 6, the killing ability of FnC-modified CAR T against leukemia cells was significantly increased, and at 1:4, the killing rate was still 80% or higher.

### 2.6 Change of peripheral blood leukemia load over time

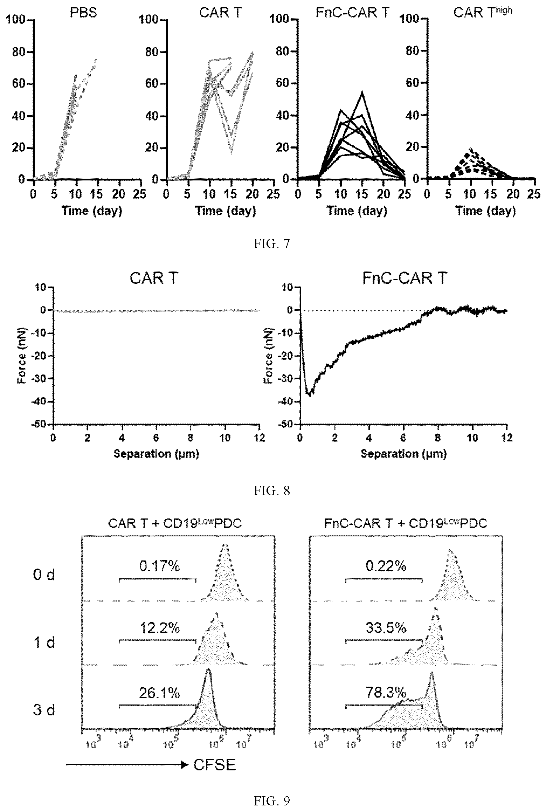

Peripheral blood leukemia load was measured as a function of time following reinfusion of CAR T, FnC-CAR T, and high dose CAR T into leukemia model mice. As shown in FIG. 7, the treatment effect was characterized by detecting the leukemia proportion in peripheral blood by sampling peripheral blood of model mice at different time points. As can be seen in FIG. 7, FnC-modified CAR T can completely inhibit the progression of leukemia and achieve nearly the same therapeutic effect as high dose CAR T.

### Example 2

### 1. Force between CAR T and FnC-CAR T and leukemia cells with low expression of CD19

The present invention further detected the force between CAR T and leukemia cells with low expression of CD19, and the force between CAR T and FnC-CAR T and leukemia cells with low expression of CD19 was detected by an atomic force microscope, as shown in FIG. 8.

### 2. Proliferation assay after incubation of CAR T and FnC-CAR T with leukemia cells with low expression of CD19

The proliferation of CAR T after co-incubation with leukemia cells with low expression of CD19 was detected in the present invention, similar to the figure of the CFSE method described above, and the results are shown in FIG. 9, which demonstrates that FnC modification promoted the effect of CAR T on leukemia cells and thus proliferation.

### 3. Activation flow cytometry of CAR T and FnC-CAR T after co-incubation with leukemia cells with low expression of CD19

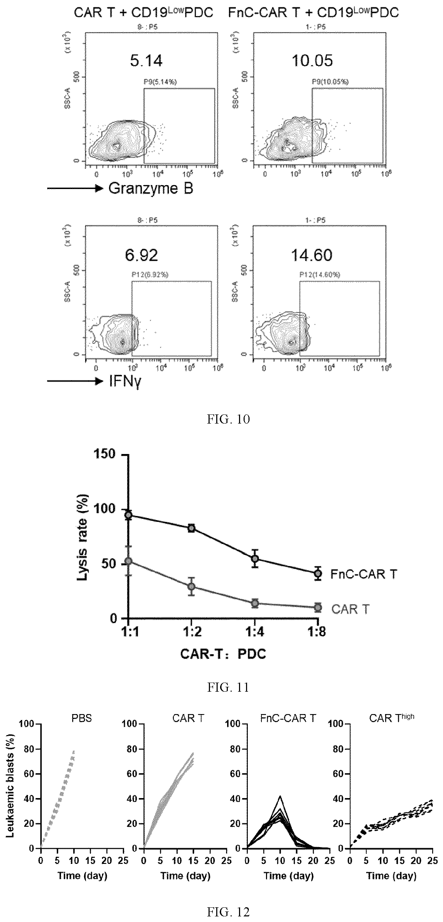

Similar to the figure of the activation flow cytometry described above, the present invention characterized activation of CAR T after co-incubation with leukemia cells with low expression of CD19, with the results shown in FIG. 10.

### 4. Killing detection of CAR T and FnC-CAR T after co-incubation with leukemia cells with low expression of CD19 according to different ratios

Similar to the figure of the LDH release killing detection, the present invention characterized the killing of leukemia cells with low expression of CD19 by CAR T, as shown in FIG. 11, which demonstrates that FnC modification enhanced the killing of leukemia cells with low expression of CD19 by CAR T.

### 5. Change of peripheral blood leukemia load over time following reinfusion of CAR T, FnC-CAR T, and high dose CAR T into model mice of leukemia cells with low expression of CD19

Similar to the figure of the *in vivo* therapeutic effect, the present invention detected the change of peripheral blood leukemia load over time following reinfusion of CAR T, FnC-CAR T, and high dose CAR T into the CD19-low expression leukemia animal model, and the results are shown in FIG. 12.

### Example 3

Labeling of cells: CAR T cells were labeled with red fluorescent Celltracker dye, leukemia cells were labeled with CFSE, and the ferritin cluster nano-particle formulation was labeled with Cy5-se dye in this experiment. The wavelengths of the three excitation lights were 561 nm, 488 nm, and 633 nm, respectively.

Specific experimental steps: The fluorescently labeled CAR T cells and fluorescently labeled ferritin cluster nano-particle formulation described above were prepared into FnC-CAR T according to the previously described method. FnC-CAR T was then blended with the above fluorescently labeled leukemia cells in a 96-well plate, and the leukemia cells were observed for FnC-CAR T surface FnC capture in real time using a high-content imaging analysis system (Operetta CLS).

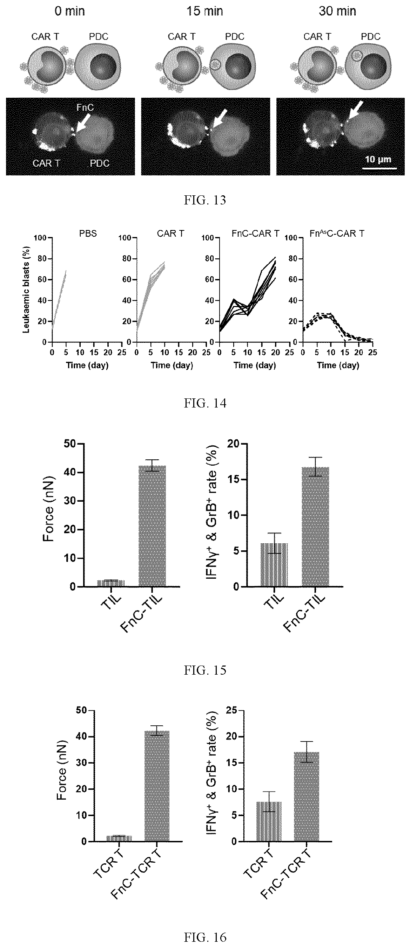

The present invention observed the capture of FnC from the surface of the CAR T by the leukemia cells when FnC-CAR T and the leukemia cells were co-incubated through the high-content imaging analysis system. The results are shown in FIG. 13, where leukemia cells were observed to capture FnC from the CAR T cell surface using a high-content dynamic fluorescence imaging system. It was clear that leukemia cells were able to take FnC off the CAR T surface while CAR T did not take up FnC.

### Example 4

The present invention detected the change of peripheral blood leukemia load over time after CAR T, FnC-CAR T, and FnC-CAR T loaded with arsenic trioxide were reinfused into high-load CD19 low-expression leukemia model mice.

The main steps of the experiment are as follows.

Construction of FnC-CAR T loaded with arsenic: Fn^{As} was constructed by first loading ferritin with the chemotherapeutic drug arsenic trioxide, and then constructing Fn^{As} into Fn^{As}C as described previously. Finally, Fn^{As}C and CAR T were constructed into Fn^{As}C-CAR T according to the foregoing method.

Evaluation of *in vivo* therapeutic effect: A CD19-low expression leukemia model was constructed using leukemia cells with low expression of CD19 as described previously. When the proportion of the human leukemia cells in the mouse peripheral blood white cells was not less than 10%, the treatment started. PBS (200 µL/mouse), CAR T (1 × 10⁶/mouse), FnC-CAR T (1 × 10⁶/mouse), and Fn^{As}C-CAR T (1 × 10⁶/mouse) were reinfused intravenously into the mice for treatment. Finally, the proportion of the human leukemia cells in the mouse peripheral blood white cells was monitored according to the method described above to detect the change of leukemia load of the mice.

The results are shown in FIG. 14, which is the same as the figure of the *in vivo* therapeutic effect of the CD19-low expression leukemia model, but the starting point of the treatment was the peripheral blood leukemia load of 10% (starting points of the two models described above were 1%).

### Example 5

The T cells used in this experiment were clinical melanoma infiltrating lymphocytes (tumor infiltrating lymphocyte, TIL). The specific steps are as follows:
Preparation of FnC-TIL: TIL used in this experiment was isolated from a tumor sample of a clinical melanoma patient, and the TIL was activated *in vitro* by using human IL-2 factor and CD3 & CD28 stimulation magnetic beads after being centrifuged and washed, and expanded. The resulting TIL was subjected to the preparation method for FnC-CAR T described above to prepare FnC-TIL using FnC and TIL.

Detection of force between TIL and tumor cells: This experiment was similar to the previous experiment, in which melanoma cell suspension was cultured in polylysine-coated 6 cm dishes, then TIL or FnC-TIL was adhered to the probe of the atomic force microscope using Cell-Tak adhesive, and the force between the two was measured using the atomic force microscope.

*In vitro* TIL activation validation: TIL and FnC-TIL were mixed with the above melanoma sample cell suspension and cultured in 96-well plates. After 72 h, TIL and FnC-TIL were characterized for activation by flow cytometry using the IFNγ and granzyme B positive ratio as described above.

The present invention further prepared FnC-TIL based on tumor infiltrating lymphocytes, and researched the force between the FnC-TIL and tumor cells and the activation condition of immune cells. The results are shown in FIG. 15, in which the left panel is the statistics of the forces between the tumor infiltrating lymphocytes and FnC-TIL with tumor cells as detected via atomic force. The right panel shows the double positive ratio of granzyme B (GrB) to interferon γ (IFNγ) after co-incubation of TIL and FnC-TIL with tumor cells to characterize the activation of TIL. In this figure, the TIL is specific to solid tumors, and the specific type of tumor is melanoma.

### Example 6

The present invention further prepared FnC-TCR T based on TCR T cells, and researched the force between the FnC-TIL and tumor cells and the activation condition of immune cells.

The T cells used in this experiment were engineered TCR T cells. The specific experiment is as follows.

Construction of FnC-TCR T: FnC and TCR T were constructed into FnC-TCR T according to the construction method of FnC-CAR T described above.

Force between TCR T and tumor cells: Human melanoma cell lines were cultured in 6 cm culture dishes. After 24 h,

TCR T or FnC-TCR T was then adhered to the probe of an atomic force microscope using Cell-Tak adhesive, and the force between TCR T or FnC-TCR T and the melanoma cells was then detected using the atomic force microscope.

*In vitro* activation validation: TCR T or FnC-TCR T was co-incubated with the melanoma cells in 96-well plates. After 72 h, TCR T and FnC-TCR T were characterized for activation by flow cytometry using the IFN-γ and granzyme B positive ratio as described above.

The results are shown in FIG. 16, in which the left panel is the statistics of the forces between the TCR T and FnC-TCR T with tumor cells as detected via atomic force. The right panel shows the double positive ratio of granzyme B (GrB) to interferon γ (IFN-γ) after co-incubation of TCR T and FnC-TCR T with tumor cells to characterize the activation of TCR T. This figure demonstrates that the potentiating effect of the FnC strategy is also effective for TCR T.

### Example 7

The present invention further prepared FnC-^{EGFR}CAR T based on EGFR CAR T (^{EGFR}CAR T) cells, and researched the force between the FnC-TIL and tumor cells and the activation condition of immune cells.

The T cells used in this experiment were human EGFR-specific CAR T (^{EGFR}CAR T). The specific experimental steps are as follows.

Preparation of FnC-^{EGFR}CAR T: FnC and ^{EGFR}CAR T were constructed into FnC-^{EGFR}CAR T according to the construction method of FnC-CAR T described above.

Detection of force between ^{EGFR}CAR T and tumor cells: Human liver cancer cell lines were cultured in 6 cm culture dishes. After 24 h, ^{EGFR}CAR T or FnC-^{EGFR}CAR T was then adhered to the probe of an atomic force microscope using Cell-Tak adhesive, and the force between ^{EGFR}CAR T or FnC-^{EGFR}CAR T and the liver cancer cells was then detected using the atomic force microscope.

*In vitro* activation validation: ^{EGFR}CAR T or FnC-^{EGFR}CAR T was co-incubated with human liver cancer cells in 96-well plates. After 72 h, ^{EGFR}CAR T and FnC-^{EGFR}CAR T were characterized for activation by flow cytometry using the IFNγ and granzyme B positive ratio as described above.

The results are shown in FIG. 17, in which the left panel is the statistics of the forces between EGFR CAR T (^{EGFR}CAR T) and FnC-^{EGFR}CAR T with tumor cells as detected via atomic force. The right panel shows the double positive ratio of granzyme B (GrB) to interferon γ (IFN-γ) after co-incubation of ^{EGFR}CAR T and FnC-^{EGFR}CAR T with tumor cells to characterize the activation of ^{EGFR}CAR T. This figure demonstrates that the potentiating effect of the FnC strategy is also effective for other kinds of CAR T.

### Example 8

This example shows the statistical results of killing of bone marrow samples from clinically different leukemia patients by different CAR T cells, and the experimental steps are as follows:
Construction of FnC-CAR T and Fn^{As}C-CAR T: The CAR T cells used in this experiment were CD7-CAR T, CD33-CAR T, and CD19-CAR T, against T-ALL, AML, and B-ALL, respectively. The corresponding FnC-CAR T and Fn^{As}C-CAR T were then constructed using the three cells according to the method described above.

Killing detection: CAR T, FnC-CAR T, and Fn^{As}C-CAR T were blended with leukemia patient bone marrow samples in a 1:1 ratio and then cultured in 96-well plates. After 24 h, the cell mixture was taken out and centrifuged, and the supernatant was taken. LDH release in the supernatant was detected using an LDH release detection kit (Solarbio), and the ratio of the leukemia cells lysed was characterized by comparison with the positive control group.

The results are shown in FIG. 19. In FIG. 19, the CAR T cells against a bone marrow sample from a T-ALL patient were CD7-CAR T cells; the CAR T cells against a bone marrow sample from an AML patient were CD33-CAR T cells; the CAR T cells against a bone marrow sample from a B-ALL patient were CD19-CAR T cells. The result shows that the targeted cell of the present invention can target the cancer cells of different types of leukemia patients and effectively kill the cancer cells of different types of leukemia patients.

### Example 9

This example shows the results of detecting the change of peripheral blood leukemia load over time after CAR T, FnC-CAR T, and high dose CAR T were reinfused into T-ALL leukemia PDX model mice. Firstly, leukemia T-ALL PDX model was constructed: Leukemia cells isolated from bone marrow of clinical T-ALL leukemia patients were reinfused intravenously into severely immunodeficient mice at a dose of 5 × 10⁶ cells/mouse (NTG mice, SPF company). After about 7 days of reinfusion, orbital blood of the mouse was sampled, and the proportion of the human leukemia cells in the white cells of the sample of the blood of the mouse was detected by flow cytometry. When the proportion was not less than 1%, it was determined that the modeling of the T-ALL PDX was successful. Treatment regimen: After the T-ALL PDX model was successfully constructed, the model mice were randomly grouped (no less than 8 mice per group), and then PBS buffer (200 µL/mouse), CAR T (1 × 10⁶/mouse), FnC-CAR T (1 × 10⁶/mouse), and high dose CAR T (5 × 10⁶/mouse) were intravenously reinfused into the model mice. After reinfusion, the proportion of the human leukemia cells in the mouse peripheral blood white cells was detected once every 5 days so as to detect the leukemia load change of the model mouse after the treatment.

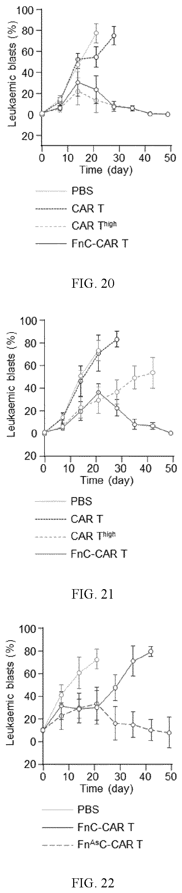

The results are shown in FIG. 20. In FIG. 20, the CAR T cells used were CD7-CAR T cells The result shows that the targeted cell of the present invention can significantly reduce the peripheral blood leukemia load of a T-ALL leukemia PDX model mouse.

### Example 10

This example shows the results of detecting the change of peripheral blood leukemia load over time after CAR T, FnC-CAR T, and high dose CAR T were reinfused into CD7 low-expression T-ALL leukemia PDX model mice. Firstly, leukemia CD7^{Low} T-ALL PDX model was constructed: Leukemia cells isolated from bone marrow of clinical T-ALL leukemia patients were down-regulated in CD7 expression using CD7 knockdown virus to construct CD7 low-expression (CD7^{Low}) leukemia cells. CD7 low-expression T-ALL cells were then reinfused intravenously into severely immunodeficient mice at a dose of 5 × 10⁶ cells/mouse (NTG mice, SPF company). After about 7 days of reinfusion, orbital blood of the mouse was sampled, and the proportion of the human leukemia cells in the white cells of the sample of the blood of the mouse was detected by flow cytometry. When the proportion was not less than 1%, it was determined that the modeling of the CD7^{Low} T-ALL PDX was successful.

Treatment regimen: After the CD7^{Low} T-ALL PDX model was successfully constructed, the model mice were randomly grouped (no less than 8 mice per group), and then PBS buffer (200 µL/mouse), CAR T (1 × 10⁶/mouse), FnC-CAR T (1 × 10⁶/mouse), and high dose CAR T (5 × 10⁶/mouse) were intravenously reinfused into the model mice. After reinfusion, the proportion of the human leukemia cells in the mouse peripheral blood white cells was detected once every 5 days so as to detect the leukemia load change of the model mouse after the treatment.

The results are shown in FIG. 21. In FIG. 21, the CAR T cells used were CD7-CAR T cells The result shows that the targeted cell of the present invention can significantly reduce the peripheral blood leukemia load of a CD7 low-expression T-ALL leukemia PDX model mouse.

### Example 11

This example shows the results of detecting the change of peripheral blood leukemia load over time after FnC-CAR T and FnAsC-CAR T were reinfused into high-load CD7 low-expression T-ALL leukemia PDX model mice. Firstly, leukemia high-load CD7^{Low} T-ALL PDX model was constructed: CD7^{Low} T-ALL cells were reinfused intravenously into severely immunodeficient mice at a dose of 5 × 10⁶ cells/mouse (NTG mice, SPF). Different time points after reinfusion, orbital blood of the mouse was sampled, and the proportion of the human leukemia cells in the white cells of the sample of the blood of the mouse was detected by flow cytometry. When the proportion was not less than 10%, it was determined that the modeling of the high-load CD7^{Low} T-ALL PDX was successful.

Treatment regimen: After the high-load CD7^{Low} T-ALL PDX model was successfully constructed, the model mice were randomly grouped (no less than 8 mice per group), and then PBS buffer (200 µL/mouse), FnC-CAR T (1 × 10⁶/mouse), and Fn^{As}C-CAR T (1 × 10⁶/mouse) were intravenously reinfused into the model mice. After reinfusion, the proportion of the human leukemia cells in the mouse peripheral blood white cells was detected once every 5 days so as to detect the leukemia load change of the model mouse after the treatment.

The results are shown in FIG. 22. In FIG. 22, the CAR T cells used were CD7-CAR T cells. The result shows that the targeted cell of the present invention can significantly reduce the peripheral blood leukemia load of a high-load CD7 low-expression T-ALL leukemia PDX model mouse.

Although the present invention has been described with reference to exemplary embodiments, it is to be understood that the present invention is not limited to the disclosed exemplary embodiments. Various modifications and variations can be made to the exemplary embodiments in the specification of the present invention without departing from the scope or spirit of the present invention. The scope of the claims should be based on the broadest interpretation to encompass all modifications and equivalent structures and functions.

## Claims

1. A T cell conjugate, comprising a T cell and a ferritin cluster, wherein the T cell binds to the ferritin cluster, and the ferritin cluster consists of ferritin.

2. The T cell conjugate according to claim 1, wherein the T cell is an activated T cell, preferably a T cell that has been expanded *in vitro.*

3. The T cell conjugate according to claim 1, wherein the ferritin cluster binds to CD71 of the T cell via receptor-ligand interaction.

4. The T cell conjugate according to claim 1, wherein the average particle size of the ferritin cluster is 50-1500 nm, preferably 300-1500 nm, further preferably 400-1000 nm, and more preferably 500-800 nm.

5. The T cell conjugate according to claim 1, wherein the ferritin cluster is formed by the aggregation and crosslinking of human ferritin;
preferably, at least 30% of the exposed ferritin subunits on the surface of the ferritin cluster are heavy chain subunits.

6. The T cell conjugate according to claim 1, wherein the T cell conjugate enhances the targeting to a CD71-expressing target cell via the ferritin cluster.

7. The T cell conjugate of claim 6, wherein the target cell comprises a tumor cell;
preferably, the target cell comprises a solid tumor cell and/or a hematological tumor cell, such as a cell from liver cancer, lung cancer, colon cancer, cervical cancer, prostate cancer, ovarian cancer, breast cancer, thyroid cancer, esophageal cancer, gastric cancer, and leukemia, preferably a leukemia cell, such as B-ALL, T-ALL, and AML.

8. A pharmaceutical composition, comprising the T cell conjugate according to any one of claims 1-7 and a pharmaceutically acceptable carrier.

9. A method for treating or ameliorating a cancer or a tumor associated with CD71 expression, comprising a step of administering to a subject in need the T cell conjugate according to any one of claims 1-7,
wherein preferably, the cancer or tumor is, for example, liver cancer, lung cancer, colon cancer, cervical cancer, prostate cancer, ovarian cancer, breast cancer, thyroid cancer, esophageal cancer, gastric cancer, or leukemia.

10. A targeted nanoparticle, wherein the targeted nanoparticle consists of a plurality of proteins interconnected via a first binding site; the targeted nanoparticle further comprises a second binding site, and binds to the outer surface of a target cell via the second binding site.

11. The targeted nanoparticle according to claim 10, wherein the targeted nanoparticle is capable of binding to the outer surface of a targeted cell.

12. The targeted nanoparticle according to claim 11, wherein the first binding site comprises an active group, and the first binding sites are linked by a linker arm.

13. The targeted nanoparticle according to claim 12, wherein the outer surface of the target cell comprises a receptor for the protein, and the receptor is capable of binding to the protein via the second binding site.

14. The targeted nanoparticle according to claim 11, wherein the targeted cell and the target cell are cells that naturally express CD71, wherein the targeted cell comprises at least one of T cells, NK cells, B cells, lymphocytes, cytokine-induced killer cells, natural lymphoid cells, and/or leukocytes.

15. The targeted nanoparticle according to claim 14, wherein the T cells comprise modified and unmodified T cells, wherein the modified T cells comprise CAR T cells and TCR T cells, and the unmodified T cells comprise tumor infiltrating T cells, PBMC-derived T cells, or other T cells isolated *in vivo.*

16. The targeted nanoparticle according to claim 10, wherein the targeted nanoparticle is 50 nm or more and 5000 nm or less.

17. The targeted nanoparticle according to claim 10, wherein the protein comprises an internal cavity.

18. The targeted nanoparticle according to claim 17, wherein the internal cavity contains or does not contain a drug.

19. The targeted nanoparticle according to any one of claims 10-18, wherein the protein is selected from at least one of natural ferritin, recombinant full-heavy chain ferritin, and genetically engineered ferritin.

20. A method for preparing the targeted nanoparticle according to any one of claim 19, comprising:
(1) aggregating and precipitating the protein to form a protein cluster under electrolyte or non-electrolyte conditions; and
(2) crosslinking the protein clusters in the presence of a crosslinking agent to obtain a crosslinked targeted nanoparticle.

21. The method for preparing the targeted nanoparticle according to claim 20, wherein the method optionally comprises a step of introducing an immunotherapeutic drug and/or a small molecule chemical drug into the cavity of the protein before step (1).

22. A targeted cell, wherein the outer surface of the targeted cell is bound to the targeted nanoparticle according to any one of claims 10-19.

23. A method for preparing the targeted cell according to claim 22, comprising:
1) aggregating and precipitating the protein to form a protein cluster under electrolyte or non-electrolyte conditions;
2) crosslinking the protein clusters in the presence of a crosslinking agent to obtain a crosslinked targeted nanoparticle; and
3) enabling the targeted nanoparticle and a cell to be close to and in contact with each other to obtain the targeted cell.

24. The method according to claim 23, wherein the method comprises a step of introducing an immunotherapeutic drug and/or a small molecule chemical drug into the cavity of the protein before step 1).

25. A method for promoting the proximity of cells to each other, comprising a step of using the T cell conjugate according to any one of claims 1-7 or the targeted nanoparticle according to any one of claims 10-19 to promote cell approach and contact, wherein a plurality of proteins are interconnected via a first binding site to form the targeted nanoparticle, the targeted nanoparticle further comprises a second binding site, and binds to the outer surface of the cell via the second binding site.

26. The method according to claim 25, wherein the surface of the cell has a receptor binding to the targeted nanoparticle or is bound to the targeted nanoparticle,
preferably, the cell is selected from at least one of CAR T cells, TCR T cells, tumor infiltrating lymphocytes, cancer cells, and tumor cells.

27. A pharmaceutical composition, comprising the targeted nanoparticle according to any one of claims 10-19, or the targeted cell according to claim 22.

28. Use of the T cell conjugate according to any one of claims 1-7, or the targeted nanoparticle according to any one of claims 10-19, or the targeted cell according to claim 22 in the manufacture of a medicament for treating or ameliorating a cancer or a tumor.
